(19) Europäisches Patentamt / European Patent Office / Office européen des brevets

(11) **EP 2 535 053 B1**

## (12) EUROPEAN PATENT SPECIFICATION

(45) Date of publication and mention
of the grant of the patent:
**15.08.2018 Bulletin 2018/33**

(21) Application number: **11739762.0**

(22) Date of filing: **02.02.2011**

(51) Int Cl.:
*A61K 35/20* (2006.01)     *A61P 3/04* (2006.01)
*A61P 3/10* (2006.01)     *A61P 43/00* (2006.01)
*A23L 33/10* (2016.01)     *A23L 33/11* (2016.01)

(86) International application number:
**PCT/JP2011/052099**

(87) International publication number:
**WO 2011/096414 (11.08.2011 Gazette 2011/32)**

(54) **MITOCHONDRIAL FUNCTION IMPROVER**

VERBESSERER DER MITOCHONDRIALEN FUNKTION

AGENT D'AMÉLIORATION DE LA FONCTION MITOCHONDRIALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2010 JP 2010022222**

(43) Date of publication of application:
**19.12.2012 Bulletin 2012/51**

(73) Proprietor: **Kao Corporation
Chuo-ku
Tokyo 103-8210 (JP)**

(72) Inventors:
• **HARAMIZU, Satoshi
Haga-gun
Tochigi 321-3497 (JP)**
• **OTA, Noriyasu
Haga-gun
Tochigi 321-3497 (JP)**
• **MURASE, Takatoshi
Haga-gun
Tochigi 321-3497 (JP)**

(74) Representative: **Vossius & Partner
Patentanwälte Rechtsanwälte mbB
Siebertstrasse 3
81675 München (DE)**

(56) References cited:
**EP-A1- 2 011 500        EP-A1- 2 039 365
WO-A1-2007/116981        WO-A1-2007/139547
WO-A1-2008/093826        JP-A- 10 231 495
JP-A- 2002 068 998        JP-A- 2002 326 932
JP-A- 2007 320 901        JP-A- 2009 500 357
JP-A- 2010 059 155**

• **HAJIME SASAKI ET AL.: 'Milk Phospholipids no
Shishitsu Taisha Chosetsu Sayo' MILK SCIENCE
vol. 51, no. 3, 2002, pages 173 - 177, XP008166334**
• **TRANSLATED BY NOBUO TAMIYA ET AL. VOET
SEIKAGAKU 01 October 1998, pages 678 - 680,
XP008166240**

**Description**

Field of the Invention

The present invention relates to nontherapeutic uses of a fat globule membrane component comprising sphingomyelin as a mitochondrial function improver, an energy consumption promoter, and a lipid combustion promoter.

Background of the Invention

Our life activities are supported by adenosine triphosphate (ATP) produced by linkage of various physical/chemical processes called metabolism. Mitochondria play a central role in energy metabolism and supply ATP by beta-oxidation of a fatty acid or oxidative phosphorylation in an electron transport system.

Oxygen consumption which reflects energy consumption in a living organism is characterized by being high in the skeletal muscle, liver, or heart. The fact corresponds to that the mitochondria are distributed at a high level in the heart muscle, liver, and skeletal muscle, indicating that the mitochondria play an important role in energy metabolism. It is said that 90% or more of oxygen consumption in a living organism are carried out in the mitochondria.

In recent years, it has been clarified that dysfunction of the mitochondria is closely related to lifestyle-related diseases, aging-related diseases, and the like. Reduction in energy metabolism due to aging is known to relate to decrease in mitochondrial functions such as a mutation or a damage of mitochondrial DNA (Non-Patent Document 1).

The decrease in mitochondrial functions causes an imbalance of energy intake and energy consumption via reduction in energy metabolism, and hence may cause lifestyle-related diseases (Non-Patent Document 2). Therefore, enhancement of the energy metabolism by maintaining/improving mitochondrial functions may lead to prevention, improvement, or reduction in risk of development of lifestyle-related diseases and may contribute to improvement of quality-of-life (QOL).

On the other hand, exercise is known to be a method of increasing the amount of mitochondria in muscle (Non-Patent Document 3). Therefore, the exercise may be considered to increase energy consumption in a living organism via an increase of mitochondria in muscle. However, although importance of exercise is widely recognized at the present day, in reality, it is difficult to carry out exercises regularly. A method of increasing energy consumption by promoting energy metabolism more effectively has been desired.

From such a viewpoint, components for enhancing a mitochondrial function and energy metabolism have been searched for.

For example, caffeine, capsaicin and the like having sympathetic nervous activating action have been reported as components for promoting energy metabolism (Non-Patent Documents 4and5) . However, caffeine and capsaicin are unsatisfactory because they have limited practical applications from the viewpoints of safety, irritant property and the like. Further examples of the components having energy metabolism promoting action include capsinoid-containing compositions (Patent Document 1) and flavans or flavanones (Patent Document 2).

Furthermore, in recent years, it has been reported that capsiate, which is a less-pungent, mild-irritant capsaicin analog, has energy metabolism promoting action (Non-Patent Document 6).

Furthermore, it has been found that tea catechin has an action of suppressing reduction in energy metabolism and deterioration of mitochondrial function due to aging (Patent Document 3). In addition, as components having mitochondrial function activating action, there are given, for example, a benzimidazole derivative or a salt thereof (Patent Document 4) and 1,2-ethanediol or a salt thereof (Patent Document 5).

However, few components for enhancing energy metabolism and mitochondrial function other than the foregoing are known.

A fat globule membrane component is a membrane that coats milk fat globules secreted from the mammary gland, and has many physiologic functions as a food for newborn animals in addition to the function of dispersing fat into milk. Examples of known physiological functions include an effect of the increase in and/or inhibiting effect of the decrease in a blood adiponectin level (Patent Document 6), a learning ability improving effect (Patent Document 7), and a sialomucin secretion promoting effect (Patent Document 8) .

However, effects of the fat globule membrane component on mitochondrial function and energy metabolism have not been known heretofore.

Prior Art Document

Patent Document

[Patent Document 1] JP-A-2004-149494
[Patent Document 2] JP-A-2007-314446
[Patent Document 3] JP-A-2008-63318
[Patent Document 4] JP-A-2004-67629
[Patent Document 5] JP-A-2002-322058
[Patent Document 6] JP-A-2007-320901
[Patent Document 7] JP-A-2007-246404
[Patent Document 8] JP-A-2007-112793

Non-Patent Document

[Non-Patent Document 1] Iwanamikouza: Gendai Igaku no Kiso, 1999 12(2): 55-58.
[Non-Patent Document 2] Ritz P. Diabetes Metab. 2005 2: 5S67-5S73.
[Non-Patent Document 3] Holloszy JO. J. Physiol. Pharmacol. 2008 59: 5-18.
[Non-Patent Document 4] Dulloo AG. Am J Clin Nutr. 1989 49(1) : 44-50.
[Non-Patent Document 5] Kawada T. Proc Soc Exp Biol Med. 1986 183(2) : 250-6.
[Non-Patent Document 6] Ohnuki K. Biosci Biotechnol Biochem. 2001 65(12): 2735-40.

Summary of the Invention

The present invention relates to the following items (1) to (4).

(1) A nontherapeutic use of a fat globule membrane component comprising sphingomyelin for mitochondrial function improvement.
(2) A nontherapeutic use of a fat globule membrane component comprising sphingomyelin for energy consumption promotion.
(3) A nontherapeutic use of a fat globule membrane component comprising sphingomyelin for lipid combustion promotion.
(4) The nontherapeutic use of the fat globule membrane component according to any one of items (1) to (3), wherein the fat globule membrane component comprises sphingomyelin from 1 to 50% by mass.

Detailed Description of the Invention

The present invention relates to providing nontherapeutic uses of a fat globule membrane component as a mitochondrial function improver, an energy consumption promoter, and a lipid combustion promoter which are derived from materials commonly consumed in diet, have high safety, and are useful in a drug, a quasi drug, a food, or a feed.
The present inventors have searched for components which are effective in improving mitochondrial function, promoting energy consumption, and promoting lipid combustion, and, as a result, have found that a fat globule membrane component has an effect of mitochondrial function improving action, energy consumption promoting action, and lipid combustion promoting action.
The mitochondrial function improver, energy consumption promoter, and lipid combustion promoter according to the present invention have high safety and excellent mitochondrial function improving action, energy consumption promoting action, and lipid combustion promoting action. Therefore, the mitochondrial function improver, energy consumption promoter, and lipid combustion promoter are useful as materials to be blended as active ingredients in foods, drinks, drugs, quasi drugs, or feeds for preventing or improving deterioration of mitochondrial function or energy metabolism.
The fat globule membrane component in the present invention is defined as a membrane which coats fat globules in milk, and a membrane component mixture that constitutes the membrane. It is known that the fat globule membrane component is contained much in a fraction which contains a high content of milk complex lipid such as butter milk and butter serum. In general, the milk fat globule membrane component is composed of about 40 to 45% by mass of proteins and about 50 to 55% by mass of lipids. It is known that the proteins include a glycoprotein called milk mucin (Mather IH, Biochim Biophys Acta. (1978) 514:25-36.) or the like, and the lipids include triglyceride and phospholipids (for example, sphingophospholipid and glycerophospholipid) in large amounts, and in addition, glycosphingolipid (for example, glucosylceramide and ganglioside) (Keenan TW, Applied Science Publishers. (1983) pp89-pp130.).
Examples of the phospholipids contained in the fat globule membrane component of the present invention include sphingophospholipids such as sphingomyelin, and, in addition, glycerophospholipids such as phosphatidylcholine and phosphatidylethanolamine. Among them, the fat globule membrane component as used in the present invention includes sphingomyelin, which is a characteristic phospholipid derived from milk.

The content of the lipids in the fat globule membrane component of the present invention is not particularly limited, but the content is preferably from 20 to 100% by mass, more preferably from 35 to 90% by mass, and even more preferably from 50 to 90% by mass in terms of dry matter.

The content of the phospholipid in the fat globule membrane component of the present invention is not particularly limited, but the content is preferably from 3 to 100% by mass, more preferably from 10 to 100% by mass, even more preferably from 15 to 85% by mass, and even more preferably 20 to 70% by mass in terms of dry matter.

The content of each phospholipid in the fat globule membrane component is not particularly limited, but, for example, the content of sphingomyelin in the fat globule membrane component is preferably from 1 to 50% by mass, more preferably from 2 to 30% by mass, even more preferably from 3 to 25% by mass, and even more preferably from 4 to 20% by mass in terms of dry matter.

As the fat globule membrane component of the present invention, components obtained from milk raw material and the like by method of preparing various fat globule membrane components, such as a centrifugation method and an organic solvent extraction method may be used. Furthermore, components whose purity is increased by purification through techniques such as dialysis, ammonium sulfate fractionation, gel filtration, isoelectric precipitation, ion-exchange chromatography, and solvent fractionation may be used.

Examples of the milk raw materials of the fat globule membrane component of the present invention include cow milk and goat milk. Among the milk, a fat globule membrane component derived from cow milk is particularly preferable because it is commonly consumed in diet, and that with high purity and low price is commercially available.

Furthermore, the milk raw materials include not only milk such as raw milk, nonfat milk, and processed milk but also dairy products. Examples of the dairy products include butter milk, butter oil, butter serum, and whey protein concentrate (WPC).

The fat globule membrane component can be prepared by a method of extracting, for example, milk, and dairy products such as whey protein concentrate (WPC) with ether or acetone (JP-A-03-47192) or a method of adjusting butter milk to an acidic range, conducting isoelectric precipitation, removing the resultant proteins, subjecting the supernatant to a microfilter membrane treatment and drying the resultant concentrate (JP-B-3103218).

Furthermore, a method of coagulating and removing proteins from butter serum, subsequently subjecting the resultant to filtration and concentration, and drying the concentrate (JP-A-2007-89535) or the like can be employed. For example, this preparation method makes it possible to prepare a fat globule membrane component containing 20% by weight or more of complex lipid derived from milk in terms of dry matter. Note here that the form of the fat globule membrane component is not particularly limited, and the form may be liquid, semisolid and solid, powdery, and the like, and these forms may be used alone or in combination of two or more thereof.

Furthermore, as the fat globule membrane component, commercially available products may be used. Examples of such commercially available products include "BSCP" produced by MEGGLE JAPAN Co., Ltd., "Milk Ceramide MC-5" produced by Snow Brand Milk Products Co., Ltd., and "Phospholipid Concentrate" produced by New Zealand Milk Products Co., Ltd.

Furthermore, since the fat globule membrane component is contained much in butter milk obtained when butter grains are produced from cream obtained by centrifugation of cow milk and the like, butter milk itself may be used. Similarly, since the fat globule membrane component is contained much in butter serum generated when butter oil is produced, butter serum itself may be used.

As described in the below-mentioned Examples, the fat globule membrane component has a mitochondrial function improving action, energy consumption promoting action, and lipid combustion promoting action.

Therefore, the fat globule membrane component can be used in a method for improving mitochondrial function, promoting energy consumption, and promoting lipid combustion by administration or intake of the fat globule membrane component in animals including humans.

Mitochondria are present in many cells in a living organism and play a particularly important role in ATP production by an oxidative phosphorylation reaction. That is to say, in the present invention, the mitochondrial function means that energy for cell survival/activity is obtained from nutrients such as carbohydrates and lipids. Furthermore, since it is thought that abnormality in the mitochondrial function is closely related to lifestyle-related diseases, aging-related diseases, and the like, the fat globule membrane component of the present invention can be used for preventing/improving insulin resistance-related diseases such as obesity and diabetes, and prostration and fatigue due to aging and inaction.

Furthermore, in the present invention, the energy consumption means that nutrients (energy sources) are metabolized in each tissue of a living organism and converted into chemical energy or thermal energy, and the energy consumption level is calculated from an amount of oxygen consumed in the process and refers to a macro physicochemical energy production level in each individual. That is to say, the energy consumption promoting action refers to an action to increase the energy consumption level defined as above. In addition, the lipid combustion means that a fatty acid is metabolized in each tissue of a living organism and converted into chemical energy or thermal energy.

The lipid combustion level is calculated from a level of oxygen consumed and a level of carbon dioxide emitted in the oxidative metabolism process by using, for example, the following equation (i) of Peronnet et al. (Peronnet et al., Can J Sport Sci. 1991 16:23-29), and refers to a level of production of energy derived from lipids in each individual. That is to say, the fat combustion promoting action refers to an action of increasing a fat combustion level defined as above.

$$\text{Lipid combustion level} = 1.695 \times (1-1.701/1.695 \times \text{respiratory quotient}) \times \text{oxygen consumption level} \quad (i)$$

$$(\text{respiratory quotient} = \text{carbon-dioxide emission level/oxygen consumption level})$$

Furthermore, the fat globule membrane component can be used as a mitochondrial function improver, an energy consumption promoter, and a lipid combustion promoter (hereinafter, referred to as "mitochondrial function improver and the like"), and can also be used for production of the mitochondrial function improver and the like. As the mitochondrial function improver and the like, the fat globule membrane component may be used alone, or, in addition, appropriately selected additives such as a carrier which are acceptable to the below-mentioned target product to incorporate the improver or the like can be used, if necessary. Note here that the preparations can be produced by conventional methods depending upon the target products to incorporate the improver or the like.

The mitochondrial function improver and the like according to the present invention can be administered to humans and animals and besides can be used as active ingredients to be blended in various foods, drinks, drugs, quasi drugs, pet foods, and the like. As foods, the mitochondrial function improver and the like may have the concept of achieving physiological functions such as improvement in mitochondrial function or promotion of energy consumption, and prevention, improvement, or reduction in risk of development of lifestyle-related diseases, and can be applied for foods and drinks, functional foods and drinks, patient foods and drinks, foods for specif ied health, and the like, to which the concept is labeled as needed.

The forms of administration of the mitochondrial function improver and the like according to the present invention used for active ingredients of drugs or quasi drugs include oral administration such as by tablets, capsules, granules, powders, and syrups, and parenteral administration such as by injections, suppositories, inhalation drugs, transdermal systems, and external preparations. Furthermore, when preparations in such various dosage forms are prepared, the mitochondrial function improver and the like according to the present invention can be used alone or appropriately in combination with a pharmaceutically acceptable excipient, binder, extender, disintegrant, surfactant, lubricant, dispersing agent, buffering agent, preservative, corrigent, flavor, coating agent, carrier, diluent, or the like. Among these forms of administration, oral administration is preferred. A liquid preparation for oral administration can be prepared by a conventional method by addition of a corrigent, a buffering agent, a stabilizing agent, and the like.

When the mitochondrial function improver and the like according to the present invention are used for active ingredients of foods, they can be used in the forms of various foods such as foods, drinks and nourishing foods. Examples thereof include cow milk, processed milk, milk beverages, yogurt, refreshing beverages, tea beverages, coffee beverages, fruit juice beverages, carbonated drink, juice, jelly, wafer, biscuits, bread, noodles, and sausage. In addition, the examples include a nutrient supplying composition having the same forms as the above-mentioned oral administration preparation (tablets, capsules, syrups, and the like).

When various forms of foods are prepared, the mitochondrial function improver and the like according to the present invention may be used alone or appropriately in combination with other food materials or a solvent, a softener, an oil, an emulsifying agent, an antiseptic, a flavor, a stabilizing agent, a colorant, an antioxidant, a moisturizing agent, a thickening agent, and the like.

Furthermore, in the case where the mitochondrial function improver and the like according to the present invention are used as active ingredients of feeds, they may be used widely in feeds for all livestock animals, and examples of the feeds include: feeds for livestock animals used for cattle, swine, poultry, sheep, horses, and goats; feeds for small animals used for rabbits, rats, and mice; feeds for fish and shellfish used for tuna, eel, sea bream, yellowtail, and shrimp; and pet foods used for dogs, cats, birds, and squirrels.

In addition to the mitochondrial function improver and the like according to the present invention, a general feed raw material such asmeats, proteins, cereals, brans, lees, saccharides, vegetables, vitamins, or minerals, or a solvent, a softener, an oil, an emulsifying agent, an antiseptic, a flavor, a stabilizing agent, a colorant, an antioxidant, a moisturizing agent, a thickening agent, or the like may be appropriately blended in the feeds to produce the feeds by a conventional method.

The mitochondrial function improver and the like according to the present invention may contain, if necessary, a medicinal ingredient appropriately selected, other than the fat globule membrane component.

The content of the fat globule membrane component (in terms of dry matter) with respect to beverages containing the mitochondrial function improver and the like according to the present invention, such as milk beverages, refreshing beverages, and tea beverages is preferably 0.001 to 5.0% by mass, more preferably 0.01 to 3.0% by mass, and even more preferably 0.1 to 2.0% by mass.

In the case of foods or feeds other than drinks, or drugs, for example, oral solid preparations such as tablets, granules, and capsules, or oral liquid preparations such as internal liquids and syrups, containing the mitochondrial function improver and the like according to the present invention, the content of the fat globule membrane component (in terms of dry matter) is preferably 0.02 to 80% by mass, more preferably 0.2 to 75% by mass, and even more preferably 2.0 to 50% by mass. Note here that the state of the fat globule membrane component is not particularly limited and may be dissolved or dispersed.

The amount of intake of the mitochondrial function improver and the like according to the present invention differs depending on the dosage forms or uses, but the daily dosage for an adult individual of the fat globule membrane component (in terms of dry matter) is preferably from 10 to 10000 mg/60 kg body weight, more preferably from 100 to 5000 mg/60 kg body weight, and even more preferably from 500 to 5000 mg/60 kg body weight. Furthermore, the mitochondrial function improver and the like can be administered in an arbitrary administration/intake regimen, and administration/intake is preferably carried out once to several times per day.

The mitochondrial function improver and the like according to the present invention are preferably administered or taken during daily activity, for example, during housekeeping or work or when commuting to school or work although the timing is not particularly limited. Furthermore, the above-mentioned preparation is administered or taken preferably three days or more per week, more preferably five days or more per week, and even more preferably every day. Furthermore, the duration of administration or intake is preferably two weeks or longer and more preferably four weeks or longer.

Subjects of administration or intake are not particularly limited as long as they are in need thereof. However, since the mitochondrial function improver according to the present invention can improve the mitochondrial function, promote energy consumption, and promote lipid combustion, they can also be administered to or taken effectively by, in particular, obese persons, persons with insulin resistance such as diabetic persons, aged persons, and persons with other mitochondrial dysfunction-related diseases (mitochondrial diseases such as Fukuhara disease, Leigh's encephalopathy, mitochondrial diabetes, Leber disease, Pearson disease, Kearns-Sayre syndrome, stroke-like episode syndrome, fatty liver diseases, and the like).

Examples

Test Example 1: Energy consumption and lipid combustion promoting action of fat globule membrane component

Evaluation on the energy consumption and lipid combustion promoting action of the fat globule membrane component was carried out as follows . As the fat globule membrane component, BSCP produced by MEGGLE JAPAN Co., Ltd. was used.

BSCP contained, in terms of drymatter, 49% by mass (hereinafter, referred to as "%") protein, 39% lipid, 3.7% sphingomyelin as a sphingophospholipid, and 2.4% glucosylceramide and 0.4% ganglioside as glycosphingolipids.

An analysis method of protein and lipid in the fat globule membrane component was carried out by the Kjeldahl method (Kandatsu Makoto, Saishin Shokuhin Bunseki-Ho (Latest Analysis of Foods), Dobunshoin) and the Roese-Gottlieb method (Japan Society for Food Engineering, Shokuhin Bunseki-Ho (Food Analysis Method), Korin Publishing Co., Ltd).

Furthermore, an analysis of phospholipid in the fat globule membrane component was carried out by an LC-MS method. That is to say, a lipid fraction was extracted from the fat globule membrane component by using chloroform/methanol (= 2:1), dried and hardened under a stream of nitrogen, and then dissolved in hexane/isopropanol (= 95:5). This sample was subjected to the below-mentioned LC-MS analysis, and phospholipid was quantified.

As specific analysis means, the followings were used. Column: Inertsil SIL 100A-3 (GL Sciences Inc., 1.5 mm $\times$ 150 mm)

    Column temperature: 40°C
    Flow rate: 0.1 mL/min
    Detector: Agilent, 1100 LC/MSD

Mobile phase: Gradient separation with solution A (hexane:isopropanol:formic acid = 95:5:0.1) and solution B (hexane:isopropanol:50 mM ammonium formate = 25:65:10)

After preliminary rearing for one week, nine-week old BALB/c mice (male: Oriental Bioservice, Inc.) were classified into two groups (eight mice in each group) such that each group had the same body weight. After that, the mice of the control group were fed with a control feed (10% lipid, 20% casein, 55.5% potato starch, 8.1% cellulose, 0.2% methionine, 2.2% vitamin (product name : Vitamin mix AIN-76, Oriental Bioservice, Inc.), and 4% mineral (product name: Mineral mix AIN-76, Oriental Bioservice, Inc.)), while the mice of the test feed group were fed with a test feed containing the fat globule membrane component (10% lipid, 20% casein, 54.5% potato starch, 8.1% cellulose, 0.2% methionine, 2.2% vitamin, 4% mineral, and 1% fat globule membrane component) each for eight weeks, and a respiratory gas analysis was carried out at the ninth week.

The mice were transferred to a chamber for respiratory gas analysis and habituated to the environment for 48 hours, and oxygen consumption level and respiratory quotient of each mouse were measured over 24 hours using Arco-2000 system (ARCOSYSTEM Inc.). As used herein, the oxygen consumption level refers to energy consumption level (mL oxygen consumption level per kg mouse body weight per min (mL/kg/min)), and the respiratory quotient refers to a ratio between carbon dioxide emission level and oxygen consumption level. From the oxygen consumption level and respiratory quotient, the lipid combustion level was calculated by an equation of Peronnet (Peronnet F, and Massicotte D (1991) Can J Sport Sci 16:23-29.). Table 1 shows average energy consumption level (mL/kg/min) and average lipid combustion level (mg/kg/min) in 24 hours.

[Table 1]

| Average oxygen consumption level in 24 hours after eight weeks of rearing | | |
|---|---|---|
| | Oxygen consumption level (mL/kg/min) | Lipid combustion level (mg/kg/min) |
| Control group | 44.8±0.8 | 4.1±0.5 |
| Test feed group | 47.0±0.8* | 6.5±0.9* |
| Statistically significant difference relative to control group: *$p$<0.05 (t-test) | | |

Table 1 shows that, in the case of the mice (test feed group) fed with the test feed containing the fat globule membrane component, the oxygen consumption level and lipid combustion level were significantly higher than those of the control feed group. Therefore, the fat globule membrane component of the present invention is useful as an energy consumption promoter, a lipid combustion promoter, or a material for those promoters.

Test Example 2: Effect of fat globule membrane component on mitochondrial function improvement

Evaluation on the energy consumption and lipid combustion promoting and mitochondrial function improving action of the fat globule membrane component was carried out as follows. As the fat globule membrane component, Phospholipid Concentrate 700 produced by New Zealand Milk Products Co., Ltd. was used.

Phospholipid Concentrate 700 contained 85% lipid and 16.5% sphingomyelin in terms of dry matter.

After preliminary rearing for one week, nine-week old BALB/c mice (male: Oriental Bioservice, Inc.) were classified into two groups (eight mice in each group) such that each group had the same body weight and swimming endurance (determined by measuring a limit swimming time by using a flowing water pool for mice (Kyodai Matsumoto type flowing water tank for measuring an amount of exercise) by the below-mentioned method).

After grouping, the mice of the control group were fed with a control feed (10% lipid, 20% casein, 55.5% potato starch, 8.1% cellulose, 0.2% methionine, 2.2% vitamin (product name: Vitamin mix AIN-76, Oriental Bioservice, Inc.), and 4% mineral (product name: Mineral mix AIN-76, Oriental Bioservice, Inc.)), and the mice of the test feed group were fed with a test feed containing the fat globule membrane component (10% lipid, 20% casein, 54.5% potato starch, 8.1% cellulose, 0.2% methionine, 2.2% vitamin, 4% mineral, and 1% fat globule membrane component) each for 13 weeks. Note here that during the period, in order to habituate the mice (control group and test feed group) to the exercise, swimming training (6 L/min, 30 min) was given twice a week. Measurement of limit swimming time: a time until a mouse was not able to swim at a flow rate of 7 L/min was measured.

After eight weeks of rearing, a respiratory gas analysis was carried out. The mice were transferred to a chamber for respiratory gas analysis and habituated to the environment for 48 hours, and the respiratory gas of each mouse was measured over 24 hours using Arco-2000 system (ARCOSYSTEM Inc.). From the oxygen consumption level (energy consumption level) and respiratory quotient of each mouse, the lipid combustion level was calculated by an equation of Peronnet (Peronnet F, and Massicotte D (1991) Can J Sport Sci 16:23-29.). Table 2 shows average energy consumption level and average lipid combustion level in 24 hours.

[Table 2]

| Average oxygen consumption level and lipid combustion level in 24 hours after eight weeks of rearing | | |
|---|---|---|
| | Oxygen consumption level (mL/kg/min) | Lipid combustion level (mg/kg/min) |
| Control group | 46.5±0.8 | 5.1±0.4 |
| Test feed group | 49.4±0.4* | 7.8±0.6* |
| Statistically significant difference relative to control group: *$p$<0.05 (t-test) | | |

Table 2 shows that, in the case of the mice fed with the test feed containing the fat globule membrane component, the oxygen consumption level and lipid combustion level were significantly higher than those of the control group. Therefore, the fat globule membrane component of the present invention is useful as an energy consumption promoter, a lipid combustion promoter, or a material for those promoters.

After 13 weeks of rearing, the gastrocnemius muscle of a mouse of each group was collected, and RNA samples were obtained using RNeasy Fibrous Tissue Mini Kit (Qiagen) . Each RNA sample was quantified, and a reverse transcription reaction was carried out in a reaction solution ($1\times$PCR buffer II (Applied Biosystems), 5 mM $MgCl_2$, 1 mM dNTP mix, 2.5 $\mu$M Oligo d[T]$_{18}$ (New England Biolabs Inc.), and 1 U/ml RNase inhibitor (TAKARA BIO INC.)) for 125 ng of RNA per reaction, to thereby obtain cDNA. The reaction was carried out under conditions of 42°C and 10 min, 52°C and 30 min, and 99°C and 5 min.

Quantitative PCR was carried out using the thus obtained cDNA as a template by ABI PRISM 7700 Sequence Detector (Applied Biosystems). The results were corrected based on the expression amount of 36B4 mRNA and represented as relative mRNA expression amounts. PGC-1$\beta$ (GenBank: NM_133249, Forward: ACGGTTTTATCACCTTCCGGT (SEQ ID NO: 1), Reverse: ATAGCTCAGGTGGAAGGAGGG (SEQ ID NO: 2)), CPT1b (GenBank: NM_009948, Forward: ACTGTTGGACATCGCCGAAC (SEQ ID NO: 3), Reverse: CCTCTTCTTCCACCAGGTGG (SEQ ID NO: 4)), and 36B4 (GenBank: NM_007475, Forward: GACATCACAGAGCAGGCCCT (SEQ ID NO: 5), Reverse: TCTCCACAGACAATGCCAGG (SEQ ID NO: 6)) were used as primers. Table 3 shows the results.

[Table 3]

| Expression of gastrocnemius muscle mitochondrial function-related gene after 13 weeks of rearing | | |
|---|---|---|
| Gene | Control group | Test feed group |
| PGC-1$\beta$ | 0.7 0±0.03 | 0.78±0.03* |
| CPT1b | 0.75±0.03 | 0.91±0.04* |
| Statistically significant difference relative to control group: *$p$<0.05 (t-test) | | |

Table 3 shows that, in the case of the mice fed with the test feed containing the fat globule membrane component, PGC-1$\beta$ and CPT1b genes related to mitochondrial biogenesis and fat combustion were significantly highly expressed in the gastrocnemius muscle compared with the control feed group. Therefore, the fat globule membrane component of the present invention is useful as a mitochondrial function improver or a material therefor.

Preparation Example

Formulation Example 1: Mitochondrial function improving and energy consumption promoting jelly food

A 0.65% mixed gelling agent of carrageenan and Locust bean gum, 5.0% concentrated fruit juice of 50% orange, 0.05% citric acid, 0.05% vitamin C, and a 1.0% fat globule membrane component (Phospholipid Concentrate 700, produced by NEW ZEALAND MILK PRODUCTS Co., Ltd.) are mixed. Water is added to the mixture so as to adjust to 100%, and the mixture is dissolved at 65°C. Furthermore, to the mixture solution, a small amount of an orange flavor is added, and the mixture solution is held for five minutes at 85°C to carry out sterilization. After that, the mixture solution is dispensed into 100 mL vessels. The mixture is allowed to stand for eight hours while it is gradually cooled to 5°C and gelled to obtain a jelly food containing the fat globule membrane component and having good solubility in the mouth, fruit flavor, and good texture.

Formulation Example 2: Mitochondrial function improving and energy consumption promoting tablet

A tablet is produced by formulation (daily dosage: 2000 mg) composed of 180 mg of ascorbic acid, 50 mg of citric acid, 12 mg of aspartame, 24 mg of magnesium stearate, 120 mg of crystalline cellulose, 274 mg of lactose, and 800 mg of a fat globule membrane component (BSCP produced by MEGGLE Japan Co., Ltd.) according to Japanese Pharmacopoeia (General Rules for Preparation: "Tablets") . Thus, tablets containing the fat globule membrane component are obtained.

Formulation Example 3: Mitochondrial function improving and energy consumption promoting yogurt

20% Skimmilk is sterilized at 120°C for 3 seconds, and inoculums of *Streptococcus thermophilus* and *Lactobacillus casei* are cultured to obtain 300 g of a yogurt base. Furthermore, 50 g of sugar, 3 g of pectin, and 5000 mg of a fat globule membrane component (Milk Ceramide MC-5, produced by Snow Brand Milk Products Co., Ltd.) are dissolved in water, and water is added so that the total amount becomes 450 g. The solution is sterilized at 120°C for 3 seconds to obtain a syrup. The above-mentioned yogurt base and syrup are mixed, and 1 g of a flavor is added thereto, followed by homogenization, to thereby obtain a mitochondrial function improving and energy consumption promoting yogurt containing the fat globule membrane component.

Formulation Example 4: Mitochondrial function improving and energy consumption promoting mayonnaise

2.8 g of salt, 0.9 g of sucrose, 0.4 g of a spice (mustard powder), 0.5 g of a seasoning (sodium glutamate), 0.5 g of a thickener, 23.0 ml of water, 8 g of a vinegar (acidity: 10%), and 3000 mg of a fat globule membrane component (Milk Ceramide MC-5, produced by Snow Brand Milk Products Co., Ltd.) are added to 16 g of egg yolk. Then, 50 g of salad oil are added thereto, and the mixture is stirred well, to thereby obtain a mitochondrial function improving and energy consumption promoting mayonnaise containing the fat globule membrane component.

Formulation Example 5: Mitochondrial function improving and energy consumption promoting oral liquid

To an appropriate amount of purified water, 1000 mg of taurine, 1000 mg of sucrose, 50 mg of caramel, 30 mg of sodium benzoate, 5 mg of vitamin B1 nitrate, 20 mg of vitamin B2, 20 mg of vitamin B6, 2000 mg of vitamin C, 100 mg of vitamin E, 2000 IU of vitamin D3, 20 mg of nicotinamide, 1000 mg of a fat globule membrane component (Milk Ceramide MC-5, produced by Snow Brand Milk Products Co. , Ltd.), 300 mg of leucine, 150 mg of isoleucine, and 150 mg of valine are added and dissolved. The mixture solution is adjusted to pH 3 with an aqueous solution of phosphoric acid. Purified water is further added so that the total amount becomes 50 mL. The resultant is sterilized at 80°C for 30 minutes to obtain a mitochondrial function improving, or energy consumption promoting beverage containing the fat globule membrane component and amino acids.

Formulation Example 6: Mitochondrial function improving and energy consumption promoting milk beverage

Purified water is added to 1.8 g of skimmilk, 2.5 g of creaming powder, 6.5 g of dextrin, 3 g of sucrose, 1.2 g of minerals, 0.3 g of vitamins, and 1.0 g of a fat globule membrane component (Phospholipid Concentrate 500, produced by NEW ZEALAND MILK PRODUCTS Co., Ltd.). The components are mixed homogeneously to obtain a mitochondrial function improving and energy consumption promoting milk beverage (100mL) containing the fat globule membrane component.

**Claims**

1.  A nontherapeutic use of a fat globule membrane component comprising sphingomyelin for mitochondrial function improvement.

2.  A nontherapeutic use of a fat globule membrane component comprising sphingomyelin for energy consumption promotion.

3.  A nontherapeutic use of a fat globule membrane component comprising sphingomyelin for lipid combustion promotion.

4.  The nontherapeutic use of the fat globule membrane component according to any one of claims 1 to 3, wherein the fat globule membrane component comprises sphingomyelin from 1 to 50% by mass.

**Patentansprüche**

1.  Nicht-therapeutische Verwendung einer Fettkügelchenmembran-Komponente, umfassend Sphingomyelin zur Verbesserung der Mitochondrienfunktion.

2.  Nicht-therapeutische Verwendung einer Fettkügelchenmembran-Komponente, umfassend Sphingomyelin zur Förderung des Energieverbrauchs.

3.  Nicht-therapeutische Verwendung einer Fettkügelchenmembran-Komponente, umfassend Sphingomyelin zur Förderung der Fettverbrennung.

4.  Nicht-therapeutische Verwendung der Fettkügelchenmembran-Komponente nach einem der Ansprüche 1 bis 3, wobei die Fettkügelchenmembran-Komponente Sphingomyelin mit einem Gew.-% von 1 bis 50 umfasst.

**Revendications**

1.  Utilisation non thérapeutique d'un composant de la membrane des globules gras comprenant de la sphingomyéline pour l'amélioration de la fonction mitochondriale.

2.  Utilisation non thérapeutique d'un composant de la membrane des globules gras comprenant de la sphingomyéline pour l'activation de la consommation d'énergie.

3.  Utilisation non thérapeutique d'un composant de la membrane des globules gras comprenant de la sphingomyéline pour l'activation de la combustion des lipides.

4.  Utilisation non thérapeutique du composant de la membrane des globules gras selon l'une quelconque des revendications 1 à 3, dans laquelle le composant de la membrane des globules gras comprend de la sphingomyéline de 1 à 50 % en masse.

## REFERENCES CITED IN THE DESCRIPTION

### Patent documents cited in the description

- JP 2004149494 A
- JP 2007314446 A
- JP 2008063318 A
- JP 2004067629 A
- JP 2002322058 A
- JP 2007320901 A
- JP 2007246404 A
- JP 2007112793 A
- JP 3047192 A
- JP 3103218 B
- JP 2007089535 A
- EP 11739762 A
- JP 2010022222 A

### Non-patent literature cited in the description

- *Iwanamikouza: Gendai Igaku no Kiso,* 1999, vol. 12 (2), 55-58
- **RITZ P.** *Diabetes Metab.,* 2005, vol. 2, 5S67-5S73
- **HOLLOSZY JO.** *J. Physiol. Pharmacol.,* 2008, vol. 59, 5-18
- **DULLOO AG.** *Am J Clin Nutr.,* 1989, vol. 49 (1), 44-50
- **KAWADA T.** *Proc Soc Exp Biol Med.,* 1986, vol. 183 (2), 250-6
- **OHNUKI K.** *Biosci Biotechnol Biochem.,* 2001, vol. 65 (12), 2735-40
- **MATHER IH.** *Biochim Biophys Acta,* 1978, vol. 514, 25-36
- **KEENAN TW.** *Applied Science Publishers,* 1983, 89, , 130
- **PERONNET et al.** *Can J Sport Sci.,* 1991, vol. 16, 23-29
- **PERONNET F ; MASSICOTTE D.** *Can J Sport Sci,* 1991, vol. 16, 23-29

SEQUENCE LISTING

<110> Kao corporation

<120> Mitochondrial function improver

<130> U2344 EP S3

<140> EP 11 73 9762.0
<141> 2011-02-02

<150> JP 2010-022222
<151> 2010-02-03

<160> 6

<170> PatentIn version 3.1

<210> 1
<211> 21
<212> DNA
<213> Artificial sequence

<220>
<223> Oligonucleotide from PGC-1\203À, as forward PCR primer

<400> 1
acggttttat caccttccgg t          21

<210> 2
<211> 21
<212> DNA
<213> Artificial sequence

<220>
<223> Oligonucleotide from PGC-1\203À, as reverse PCR primer

<400> 2
atagctcagg tggaaggagg g          21

<210> 3
<211> 20
<212> DNA
<213> Artificial sequence

<220>
<223> Oligonucleotide from CPT1b, as forward PCR primer

<400> 3
actgttggac atcgccgaac          20

<210> 4
<211> 20
<212> DNA
<213> Artificial sequence

<220>
<223> Oligonucleotide from CPT1b, as reverse PCR primer

<400> 4
cctcttcttc caccaggtgg          20

<210> 5
<211> 20
<212> DNA
<213> Artificial sequence

<220>
<223> Oligonucleotide from 36B4, as forward PCR primer

<400> 5
gacatcacag agcaggccct          20

<210> 6
<211> 20
<212> DNA
<213> Artificial sequence

<220>
<223> Oligonucleotide from 36B4, as reverse PCR primer

<400> 6
tctccacaga caatgccagg          20